(19) Europäisches Patentamt
European Patent Office
Office européen des brevets

(11) Veröffentlichungsnummer: **0 189 060**
**A2**

# (12) EUROPÄISCHE PATENTANMELDUNG

(21) Anmeldenummer: **86100208.7**

(22) Anmeldetag: **09.01.86**

(51) Int. Cl.⁴: **A 61 K 31/44**, C 07 D 211/90

(30) Priorität: **19.01.85 DE 3501695**

(43) Veröffentlichungstag der Anmeldung: **30.07.86**
**Patentblatt 86/31**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Wehinger, Egbert, Dr., Gellertweg 33, D-5600 Wuppertal 1 (DE)**
Erfinder: **Towart, Robertson, Dr., 13 Sefton Paddock, Stoke Poges, Sl 2 4 PT (GB)**

(54) **Verwendung von Sulfonyl-dihydropyridinen als Arzneimittel zur Behandlung von Asthma.**

(57) Die vorliegende Erfindung betrifft die Verwendung von teilweise bekannten Sulfonyl-1,4-dihydropyridinen der allgemeinen Formel I

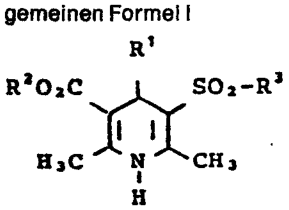

(I)

in welcher $R^1$, $R^2$ und $R^3$ die in der Beschreibung angegebene Bedeutung haben, bei der Bekämpfung von Erkrankungen der Atemwege, insbesondere ihre Verwendung in Arzneimitteln mit antiasthmatischer Wirkung.

0 189 060

BAYER AKTIENGESELLSCHAFT 5090 Leverkusen, Bayerwerk

Konzernverwaltung RP KS/by-c
Patentabteilung II(Pha)

# Verwendung von Sulfonyl-dihydropyridinen als Arzneimittel zur Behandlung von Asthma

Die vorliegende Erfindung betrifft die Verwendung von teilweise bekannten Sulfonyl-1,4-dihydropyridinen bei der Bekämpfung von Erkrankungen der Atemwege, insbesondere ihre Verwendung in Arzneimitteln mit antiasthmatischer Wirkung.

Es ist bereits bekannt, daß Sulfonyl-1,4-dihydropyridine eine gefäßerweiternde Wirkung besitzen und als Coronartherapeutika und antihypertensiv wirksame Mittel eingesetzt werden können (vgl. Deutsche Offenlegungsschriften 25 24 277, 26 16 995, 26 39 498).

Weiterhin ist bekannt, daß einige 1,4-Dihydropyridin-Derivate protektiv gegen verschiedenartig induzierte Bronchialkontraktionen wirksam sind (P.J. Barnes, Thorax 38, 481 (1983)).

Die nunmehr aufgefundene, bevorzugte Wirkung von 5-Sulfonyl-1,4-dihydropyridinen auf die Bronchialmuskulatur

ist jedoch neu und war nach Kenntnis des Standes der Technik nicht zu erwarten.

Es wurde gefunden, daß 5-Sulfonyl-1,4-dihydropyridine der allgemeinen Formel (I)

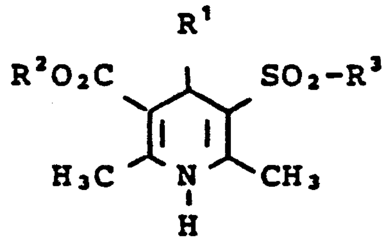

(I)

in welcher

$R^1$ für Aryl mit 6 oder 10 C-Atomen steht, gegebenenfalls substituiert durch 1 bis 3 gleiche oder verschiedene Substituenten aus der Reihe: Halogen, Cyano, Nitro, Trifluormethyl, Trifluormethoxy, Alkoxycarbonyl (bis 4 C-Atome), Carboxy, Alkoxy (bis 4 C-Atome) oder geradkettiges oder verzweigtes Alkyl mit bis zu 4 C-Atomen,

oder

für Heteroaryl steht,

$R^2$ für geradkettiges, verzweigtes oder cyclisches, gesättigtes oder ungesättigtes Alkyl mit bis zu 7 C-Atomen steht, das durch 1 bis 2 Sauerstoff- und/oder Schwefelatome in der Kette unterbrochen

sein kann und das gegebenenfalls substituiert ist durch Halogen, Nitro, Cyano, Carboxy, Alkoxycarbonyl (bis 4 C-Atome), Aryl (6, 10 C-Atome), oder eine Aminogruppe, wobei die Aminogruppe einen oder zwei gleiche oder verschiedene Substituenten aus der Gruppe Alkyl (bis 4 C-Atome), Aryl (6 bis 10 C-Atome) oder Aralkyl (7 bis 10 C-Atome) tragen kann,

$R^3$ für Aryl (6, 10 C-Atome) steht, das gegebenenfalls substituiert sein kann durch 1 bis 3 gleiche oder verschiedene Substituenten aus der Reihe: Halogen, Cyan, Nitro, Trifluormethyl, Trifluormethoxy, Alkoxy (bis 4 C-Atome), Dialkylamino, oder

für geradkettiges, verzweigtes oder cyclisches Alkyl mit bis zu 8 C-Atomen steht, das gegebenenfalls substituiert sein kann durch Cyan, Halogen oder Nitro,

zur Verwendung als Arzneimittel gegen Asthma besonders gut geeignet sind.

Bevorzugt sind Verbindungen der allgemeinen Formel (I), in welcher

$R^1$ für Phenyl steht, das gegebenenfalls substituiert sein kann durch 1 bis 2 gleiche oder verschiedene

Substituenten aus der Reihe: Fluor, Chlor, Brom, Nitro, Cyan, Trifluormethyl, Trifluormethoxy, Alkoxy (bis 2 C-Atome), Alkyl (bis 2 C-Atome),

oder

für Pyridyl, Thienyl, Furyl oder Benzoxadiazolyl steht,

$R^2$ für geradkettiges, verzweigtes oder cyclisches, gesättigtes oder ungesättigtes Alkyl mit bis zu 6 C-Atomen steht, das gegebenenfalls durch 1 bis 2 Sauerstoff- und/oder Schwefelatome in der Kette unterbrochen sein kann und das gegebenenfalls substituiert sein kann durch ein bis drei gleiche oder verschiedene Substituenten aus der Gruppe Fluor, Chlor, Brom, Cyano, Nitro, Alkoxycarbonyl (bis zu 2 C-Atomen), Phenyl oder eine Aminogruppe, wobei die Aminogruppe durch einen oder zwei gleiche oder verschiedene Substituenten aus der Reihe: Alkyl (bis 2 C-Atome), Benzyl substituiert sein kann

und

$R^3$ für Phenyl steht, das gegebenenfalls substituiert sein kann durch 1 bis 2 gleiche oder verschiedene Substituenten aus der Reihe: Fluor, Chlor, Brom, Nitro, Trifluormethyl, Cyano, Trifluormethoxy, Alkyl (bis 2 C-Atome),

oder

für geradkettiges, verzweigtes oder cyclisches Alkyl (bis 7 C-Atome) steht, das gegebenenfalls durch Fluor, Chlor, Brom, Cyano oder Nitro substituiert sein kann.

Besonders bevorzugt sind Verbindungen der allgemeinen Formel (I), in welcher

$R^1$ für gegebenenfalls durch 1 bis 2 Chlor, Cyano, Nitro oder Trifluormethyl substituiertes Phenyl steht

oder

für Pyridyl steht,

$R^2$ für geradkettiges oder verzweigtes Alkyl mit bis zu 5 C-Atomen steht, das gegebenenfalls durch ein Sauerstoff- und/oder Schwefelatom unterbrochen sein kann, und das gegebenenfalls durch ein, zwei oder drei Substituenten aus der Gruppe Fluor, Cyano oder die Benzyl-methyl-aminogruppe substituiert sein kann,

und

$R^3$ für gegebenenfalls durch 1 bis 2 Chlor, Nitro, Cyano, Trifluormethyl substituiertes Phenyl steht

oder

für Methyl oder Ethyl, wobei Ethyl gegebenenfalls durch ein bis drei Fluor substituiert sein kann,

zur Verwendung als Arzneimittel gegen Asthma.

Für die Verwendung gegen Asthma eignen sich insbesondere die Stoffe 1,4-Dihydro-2,6-dimethyl-5-phenylsulfonyl-4-(2-trifluormethylphenyl)-pyridin-3-carbonsäureethylester, 1,4-Dihydro-2,6-dimethyl-5-methylsulfonyl-4-(3-nitrophenyl)-pyridin-3-carbonsäure-(2-ethylthio)ethylester und 1,4-Dihydro-2,6-dimethyl-5-methylsulfonyl-4-(2-nitrophenyl)-pyridin-3-carbonsäuremethylester.

Die erfindungsgemäßen Stoffe wirken bereits nach Applikation geringer Dosen selektiv auf die Bronchialmuskulatur.

Aufgrund ihrer speziellen Eigenschaften sind sie insbesondere zur Behandlung von Asthmapatienten geeignet und stellen somit eine Bereicherung der Pharmazie dar.

Die erfindungsgemäßen Stoffe sowie einige Verfahren zu ihrer Herstellung sind teilweise bekannt (vgl. Deutsche Offenlegungsschriften 25 24 277, 26 16 995, 26 39 498). Sie werden bevorzugt hergestellt durch Umsetzung von Ylidenverbindungen der Formel (II)

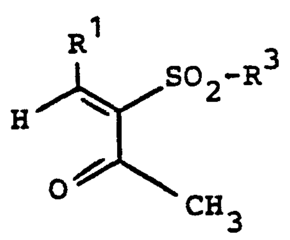

(II)

in welcher

$R^1$ und $R^3$ die oben angegebenen Bedeutungen haben,

mit Aminocrotonsäureestern der allgemeinen Formel (III)

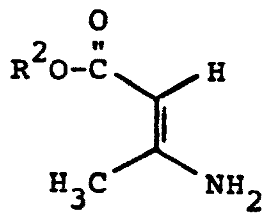

(III)

in welcher

$R^2$ die oben angegebene Bedeutung hat,

in Gegenwart von inerten Lösungsmitteln, insbesondere von niederen aliphatischen Alkoholen und von Dimethylformamid, Dimethylsulfoxid, Acetonitril oder Hexamethylphosphorsäuretriamid bei Temperaturen zwischen 10° und 160°C, insbesondere zwischen 20° und 120°C umsetzt. Die Umsetzung erfolgt gegebenenfalls unter Inertgasatmosphäre, z.B. unter Stickstoff.

Die Verbindungen können in bekannter Weise in die üblichen Formulierungen überführt werden wie Tabletten, Kapseln, Dragees, Granulate, Sirupe, Emulsionen, Suspensionen, Lösungen und insbesondere Inhalate, Sprays oder Aerosole unter Verwendung inerter, nicht-toxischer, pharmazeutisch geeigneter Trägerstoffe, Lösungs- oder Treibmitteln. Hierbei soll die therapeutisch wirksame

Verbindung jeweils in einer Konzentration von 0,5 bis 90 Gew.-% der Gesamtmischung vorhanden sein, um die erforderliche Dosierung zu erreichen. Gegebenenfalls kann auch die Reinsubstanz inhaliert werden.

Bei parenteraler Applikation werden vorzugsweise Mengen von 0,001 bis 1 mg/kg, insbesondere 0,001 bis 0,1 mg/kg Körpergewicht zur Erziehung wirksamer Ergebnisse verabreicht. Bei oraler Applikation beträgt die Dosierung vorzugsweise 0,01 bis 10 mg/kg, insbesondere 0,1 bis 5 mg/kg Körpergewicht pro Tag.
Erforderlichenfalls kann von den genannten Mengen abgewichen werden und zwar in Abhängigkeit vom Körpergewicht und der speziellen Art des Applikationsweges.

Auch der Zeitpunkt der Verabreichung und das Interwall zwischen einzelnen Verabreichungen spezieller Formulierungen kann eine Veränderung der Dosierung bedingen. So kann es sowohl ausreichend sein, mit weniger als der genannten Mindestmenge auszukommen, als auch notwendig sein, in anderen Fällen die obere Grenze der genannten Mengen zu überschreiten.

Die erfindungsgemäß verwendbaren Mittel werden beispielsweise hergestellt durch Verstrecken des Wirkstoffes mit Lösungsmitteln, Trägerstoffen und/oder Treibgasen, gegebenenfalls unter Verwendung von Emulgiermitteln und/oder Dispergiermitteln.

Als Hilfsstoffe seien beispielhaft aufgeführt: Wasser, nicht-toxische organische Lösungsmittel, wie Paraffine (z.B. Erdölfraktionen), pflanzliche Öle (z.B. Erdnuß-Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. hochdisperse Kieselsäure, Silikate), Zucker (z.B. Roh-, Milch- und Traubenzucker); Emulgiermittel, wie nicht-ionogene und anionische Emulgatoren (z.B. Polyoxyethylen-Fettsäureester, Polysulfonate), Dispergiermittel (z.B. Lignin, Sulfitablaugen, Methylzellulose, Stärke und Polyvinylpyrrolidon) und Gleitmittel (z.B. Magnesiumstearat, Talkum, Stearinsäure und Natriumlaurylsulfat, Treibgase (z.B. Frigene, Stickstoff, Lachgas, Propan, Butan oder $CO_2$).

Die Applikation erfolgt in üblicher Weise, vorzugsweise enteral oder parenteral, insbesondere durch Inhalation.

Im Fall der enteralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendung gedacht sind, können die Wirkstoffe mit verschiedenen Geschmacksaufbesserern oder Farbstoffen versetzt werden.

Für den Fall der parenteralen Anwendung können Lösungen des Wirkstoffes unter Verwendung geeigneter flüssiger Trägermaterialien eingesetzt werden. Zur Inhalation können die Wirkstoffe sowohl als Reinsubstanz als auch als Lösung unter Verwendung geeigneter Treibgase eingesetzt werden.

Überraschenderweise zeigen die erfindungsgemäß verwendeten Stoffe in biologischen Tests bereits bei geringen Dosen Relaxation der kontrahierten Bronchialmuskulatur, ohne daß die cardiovaskulären Wirkungen auftreten, wie es bei anderen Dihydropyridinen der Fall ist.

Die Wirkung der erfindungsgemäß verwendeten Stoffe zeigen beispielhaft die folgenden Testergebnisse mit 1,4-Dihydro-2,6-dimethyl-5-methylsulfonyl-4-(2-nitrophenyl)-pyridin-3-carbonsäuremethylester (im weiteren A genannt):

1) Studien an isolierten Tracheenringen normaler Meerschweinchen:
Die Carbachol-induzierte Kontraktion der Tracheenringe von Meerschweinchen werden konzentrationsabhängig durch Stoff A gehemmt. Der $IC_{50}$-Wert beträgt $1,1 \cdot 10^{-7}$ g/ml.

2) Studien am isolierten Human-Bronchialmuskel:
Isolierte Human-Bronchialmuskeln werden mit $10^{-7}$ g/ml Carbachol kontrahiert. Die Zugabe von Stoff A reduziert konzentrationsabhängig die Kontraktion. Der $IC_{50}$-Wert beträgt $6 \cdot 10^{-8}$ g/ml.

Patentansprüche

1. 5-Sulfonyl-1,4-dihydropyridine der allgemeinen Formel (I)

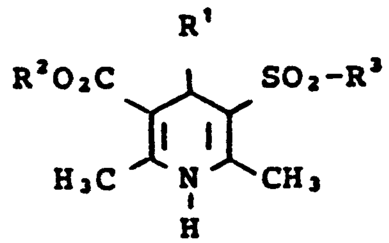

(I)

in welcher

$R^1$ für Aryl mit 6 oder 10 C-Atomen steht, gegebenenfalls substituiert durch 1 bis 3 gleiche oder verschiedene Substituenten aus der Reihe: Halogen, Cyano, Nitro, Trifluormethyl, Trifluormethoxy, Alkoxycarbonyl (bis 4 C-Atome), Carboxy, Alkoxy (bis 4 C-Atome) oder geradkettiges oder verzweigtes Alkyl mit bis zu 4 C-Atomen,

oder

für Heteroaryl steht,

$R^2$ für geradkettiges, verzweigtes oder cyclisches, gesättigtes oder ungesättigtes Alkyl mit bis zu 7 C-Atomen steht, das durch 1 bis 2 Sauerstoff- und/oder Schwefelatome in der Kette unterbrochen sein kann und das

gegebenenfalls substituiert ist durch Halogen, Nitro, Cyano, Carboxy, Alkoxycarbonyl (bis 4 C-Atome), Aryl (6, 10 C-Atome), oder eine Aminogruppe, wobei die Aminogruppe einen oder zwei gleiche oder verschiedene Substituenten aus der Gruppe Alkyl (bis 4 C-Atome), Aryl (6 bis 10 C-Atome) oder Aralkyl (7 bis 10 C-Atome) tragen kann,

$R^3$ für Aryl (6, 10 C-Atome) steht, das gegebenenfalls substituiert sein kann durch 1 bis 3 gleiche oder verschiedene Substituenten aus der Reihe: Halogen, Cyan, Nitro, Trifluormethyl, Trifluormethoxy, Alkoxy (bis 4 C-Atome), Dialkylamino, oder

für geradkettiges, verzweigtes oder cyclisches Alkyl mit bis zu 8 C-Atomen steht, das gegebenenfalls substituiert sein kann durch Cyan, Halogen oder Nitro,

zur Verwendung bei der Bekämpfung von asthmatischen Erkrankungen.

2. Verbindungen der allgemeinen Formel (I) gemäß Anspruch 1, in welcher

$R^1$ für Phenyl steht, das gegebenenfalls substituiert sein kann durch 1 bis 2 gleiche oder

verschiedene Substituenten aus der Reihe: Fluor, Chlor, Brom, Nitro, Cyan, Trifluormethyl, Trifluormethoxy, Alkoxy (bis 2 C-Atome), Alkyl (bis 2 C-Atome),

oder

für Pyridyl, Thienyl, Furyl oder Benzoxadiazolyl steht,

$R^2$ für geradkettiges, verzweigtes oder cyclisches, gesättigtes oder ungesättigtes Alkyl mit bis zu 6 C-Atomen steht, das gegebenenfalls durch 1 bis 2 Sauerstoff- und/oder Schwefelatome in der Kette unterbrochen sein kann und das gegebenenfalls substituiert sein kann durch ein bis drei gleiche oder verschiedene Substituenten aus der Gruppe Fluor, Chlor, Brom, Cyano, Nitro, Alkoxycarbonyl (bis zu 2 C-Atomen), Phenyl oder eine Aminogruppe, wobei die Aminogruppe durch einen oder zwei gleiche oder verschiedene Substituenten aus der Reihe: Alkyl (bis 2 C-Atome), Benzyl substituiert sein kann

und

$R^3$ für Phenyl steht, das gegebenenfalls substituiert sein kann durch 1 bis 2 gleiche oder verschiedene Substituenten aus der Reihe: Fluor, Chlor, Brom, Nitro, Trifluormethyl,

Cyano, Trifluormethoxy, Alkyl (bis 2 C-Atome),

oder

für geradkettiges, verzweigtes oder cyclisches Alkyl (bis 7 C-Atome) steht, das gegebenenfalls durch Fluor, Chlor, Brom, Cyano oder Nitro substituiert sein kann,

zur Verwendung bei der Bekämpfung von asthmatischen Erkrankungen.

3. Verbindungen der allgemeinen Formel (I) gemäß Anspruch (I), in welcher

$R^1$ für gegebenenfalls durch 1 bis 2 Chlor, Cyano, Nitro oder Trifluormethyl substituiertes Phenyl steht

oder

für Pyridyl steht,

$R^2$ für geradkettiges oder verzweigtes Alkyl mit bis zu 5 C-Atomen steht, das gegebenenfalls durch ein Sauerstoff- und/oder Schwefelatom unterbrochen sein kann, und das gegebenenfalls durch ein, zwei oder drei Substituenten aus der Gruppe Fluor, Cyano oder die Benzylmethyl-aminogruppe substituiert sein kann,

und

$R^3$ für gegebenenfalls durch 1 bis 2 Chlor, Nitro, Cyano, Trifluormethyl substituiertes Phenyl steht

oder

für Methyl oder Ethyl, wobei Ethyl gegebenenfalls durch ein bis drei Fluor substituiert sein kann,

zur Verwendung bei der Bekämpfung von asthmatischen Erkrankungen.

4. Verwendung von Verbindungen der allgemeinen Formel (I) gemäß Anspruch 1 zur Bekämpfung von asthmatischen Erkrankungen.

5. Verwendung von Verbindungen der allgemeinen Formel (I) gemäß Anspruch 1 zur Herstellung eines Mittels mit antiasthmatischer Wirkung.

6. Antiasthmatikum enthaltend mindestens eine Verbindung aus der allgemeinen Formel (I) gemäß Anspruch 1.

7. Antiasthmatikum gemäß Anspruch 6 in oraler Zubereitungsform enthaltend den Wirkstoff in einer Dosierung von 0,01 bis 10 mg/kg Körpergewicht.

8. Verfahren zur Herstellung eines Antiasthmatikums, dadurch gekennzeichnet, daß man Verbindungen der allgemeinen Formel (I) gemäß Anspruch 1 gegebenenfalls unter Verwendung von Hilfs- und Trägerstoffen in eine geeignete Applikationsform überführt.

9. Verfahren zur Herstellung von antiasthmatischen Arzneimitteln gemäß Anspruch 1, dadurch gekennzeichnet, daß man Ylidenverbindungen der allgemeinen Formel (II)

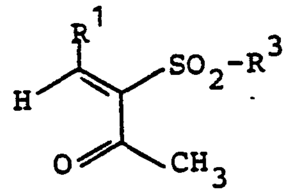

(II)

in welcher

$R^1$ und $R^3$ die oben angegebenen Bedeutungen haben,

mit Aminocrotonsäureestern der allgemeinen Formel (III)

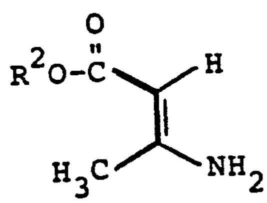

(III)

in welcher

$R^2$ die oben angegebene Bedeutung hat,

in Gegenwart von inerten Lösungsmitteln bei Temperaturen zwischen 10° und 160°C umsetzt, und die so erhaltene Verbindung unter Verwendung von Hilfs- und Trägerstoffen in eine geeignete Applikationsform überführt.